# EUROPEAN PATENT SPECIFICATION

(11) **EP 4 021 497 B1**
(45) Date of publication and mention of the grant of the patent: **25.02.2026**
(21) Application number: 20858260.1
(22) Date of filing: 29.08.2020
(51) Int. Cl.: A61K 39/395, A61K 9/00, A61K 47/10, A61K 47/18, A61K 47/02, A61K 39/00, C07K 16/42, A61K 47/26

(54) **NOVEL FORMULATION OF HIGHLY CONCENTRATED PHARMACOLOGICALLY ACTIVE ANTIBODY**
NEUARTIGE FORMULIERUNG EINES HOCHKONZENTRIERTEN PHARMAKOLOGISCH AKTIVEN ANTIKÖRPERS
NOUVELLE FORMULATION D'ANTICORPS PHARMACOLOGIQUEMENT ACTIF HAUTEMENT CONCENTRÉ

(30) Priority: 30.08.2019 IN 201921035059
(43) Date of publication of application: 06.07.2022
(73) Proprietor: Kashiv Biosciences, LLC, Piscataway, NJ 08854 (US)
(72) Inventor: WEAVER, Bruce, Piscataway, New Jersey 08854 (US); NARAYAN, Om, Gujarat Ahmedabad 382210 (IN); SHAH, Sumit Maheshkumar, Gujarat Ahmedabad 382210 (IN)
(74) Representative: Haseltine Lake Kempner LLP
(86) International application number: PCT/IB2020/058080
(87) International publication number: WO 2021/038532

(56) References cited:
- EP-A2- 2 116 265
- WO-A1-2013/096791
- US-A1- 2004 197 324
- US-A1- 2017 326 069
- US-B2- 10 080 793
- US-B2- 9 382 317
- ANN L DAUGHERTY AND RANDALL J MRSNY ED - STEVEN J SHIRE ET AL: "Formulation and Delivery Issues for Monoclonal Antibody Therapeutics", 1 January 2010, CURRENT TRENDS IN MONOCLONAL ANTIBODY DEVELOPMENT AND MANUFACTURING, SPRINGER, US, PAGE(S) 103 - 129, ISBN: 978-0-387-76642-3, XP009133774
- WANG W ET AL: "ANTIBODY STRUCTURE, INSTABILITY, AND FORMULATION", JOURNAL OF PHARMACEUTICAL SCIENCES, WILEY, vol. 96, no. 1, 1 January 2007 (2007-01-01), pages 1 - 26, XP009084505, ISSN: 0022-3549, DOI: 10.1002/JPS.20727

## Description

### FIELD OF THE INVENTION

The present invention relates to novel liquid formulations comprising high concentrated pharmacologically active antibody. The present invention further provides stable liquid formulations comprising pharmacologically active antibody having low aggregation, viscosity and low osmolarity.

### BACKGROUND OF THE INVENTION

In earlier days antibody formulations were stabilised with lyophilisation of antibody and were reconstituted prior to use with appropriate solvent system. However, with time, the requirement of stable high concentrated liquid formulation is increased because it can be administrated subcutaneously in shorter time compared to intravenous injections. Further, patient can administer subcutaneously at home which avoids clinic visits. However, high concentrated antibody formulation comprises high amount of antibody approximately more than 100mg/ml in small volume which leads to protein-protein interaction and increased viscosity. Viscosity is not only an issue regarding the biophysical and biochemical properties of the therapeutic protein, but also for the delivery and manufacturing of such highly concentrated protein solutions. Higher the viscosity of the solution the longer it takes to inject such a viscous solution via syringe and needle. In addition, protein-protein interaction in high concentrated antibody formulation tends to form oligomerization, aggregation and thereby makes unstable formulation. It has been noted that problems such as aggregation, viscosity, fragmentation, insolubility and degradation of antibodies normally increases as concentration of antibody increases in formulations. Further, aggregation of antibodies affects their stability in storage, including shelf-life. It has been well recognised that there is no standard formulation strategy which can work for all types of antibodies to provide stable and concentrated solution for pharmaceutical use.

It is well established in the prior art that protein formulation can be stabilised with use of particular pharmaceutical excipient. It is the prime goal of pharmaceutical formulator to prepare a formulation with highest stability which can provide antibody in its desired form throughout shelf-life of product.

US Patent 10034940 disclosed highly concentrated formulations of antibodies, which are particularly suitable for subcutaneous administration. Further, it covers formulation of omalizumab with different excipients like arginine-HCl, histidine and polysorbate.

US Patent 8703126 disclosed method of reducing the viscosity of a formulation of monoclonal antibody with buffer is derived from arginine or histidine.

Based on the increased demand in the concentrated liquid formulation comprising antibodies, there is a need to prepare stable liquid formulations comprising high concentrated pharmacologically active antibody.

### SUMMARY OF THE INVENTION

The present invention relates to novel stable liquid formulations comprising highly concentrated pharmacologically active antibody as defined in the claims and process for preparation of the same

The present disclosure provides a pharmaceutical stable liquid formulation comprising:
a. pharmacologically active antibody which binds to IgE;
b. phosphate buffer;
c. suitable aggregation inhibitor selected from Arginine or suitable salt thereof;
d. suitable surfactant and
e. pH 6.0 to 7.0

wherein the antibody concentration is at least 100 mg/ml,
wherein the pharmacologically active antibody which binds to IgE is Omalizumab, and
wherein the surfactant is Poloxamer 188.

In one example which is not present in the wording of the claims, the present disclosure provides pharmaceutical liquid formulation comprising:
a. 100 to 160 mg/ml of Omalizumab antibody;
b. 5mM to 20mM phosphate buffer;
c. 100mM to about 200mM of Lysine or Lysine HCl as an aggregation inhibitor;
d. 0.02% to about 0.04% of Poloxamer 188;
e. pH 6.0

In another aspect, the present disclosure provides pharmaceutical liquid formulation comprising:
a. 100 to 160 mg/ml of Omalizumab antibody;
b. 5mM to 20mM phosphate buffer;
c. 100mM to about 200mM of Arginine or Arginine HCl as an aggregation inhibitor;
d. 0.02% to about 0.04% of Poloxamer 188;
e. pH 6.0

In one example which is not present in the wording of the claims, the present disclosure provides pharmaceutical liquid formulation comprising:
a. pharmacologically active antibody which binds to IgE;
b. histidine buffer;
c. Lysine or suitable salt thereof as an aggregation inhibitor;
d. suitable surfactant and
e. pH 6.0 to 7.0
Wherein the antibody concentration is at least 100 mg/ml.

The present disclosure provides a method of manufacturing a liquid pharmaceutical composition, which is not present in the wording of the claims, wherein the method comprises mixing together Omalizumab, a phosphate buffer and an aggregation inhibitor selected from Arginine or Lysine or salts thereof, and a surfactant. Also provided is a liquid pharmaceutical composition obtainable by, obtained by, or directly obtained by a method of manufacturing a liquid pharmaceutical composition as defined herein.

In another aspect, the present disclosure provides a drug delivery device (e.g. pre-filled syringe or pen, or intravenous bag) comprising a liquid pharmaceutical composition as defined herein.

In another aspect, the present disclosure provides stable pharmaceutical liquid formulations comprising high monomer and less aggregates, fragmentation.

### DETAILED DESCRIPTION OF THE PREFERRED EMBODIMENT

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Unless otherwise defined, all technical and scientific terms used herein have the same meaning as commonly understood by one of ordinary skill in the art to which this invention pertains. In the case of conflict, the present document, including definitions will control. The term "Around," "about" or "approximately" shall generally mean within 20 percent, within 10 percent, within 5, 4, 3, 2 or 1 percent of a given value or range. Numerical quantities given are approximate, meaning that the term "around," "about" or "approximately" can be inferred if not expressly stated.

The term "Omalizumab" or "Omalizumab monomer" or "monomer" is equivalent to Omalizumab. Omalizumab is a recombinant DNA-derived humanized IgGlK monoclonal antibody that selectively binds to human immunoglobulin (IgE). The antibody has a molecular weight of approximately 149 kD. Xolair is produced by a Chinese hamster ovary cell. For the purpose of the present application, the term "Omalizumab" used herein comprises similar properties to marketed Xolair but not limited to concentrated formulations, injectable ready-to-use formulations; formulations reconstituted with water, alcohol, and/or other ingredients, and others.

As used herein, the terms "acidic variant" or "acidic species" and "AV" refer to the variants of a protein, e.g., an antibody or antigen-binding portion thereof, which are characterized by an overall acidic charge. For example, in monoclonal antibody (mAb) preparations, such acidic species can be detected by various methods, such as ion exchange, for example, WCX this is HPLC (a weak cation exchange chromatography), or IEF (isoelectric focusing). Acidic variants of antibodies are formed through chemical and enzymatic modifications such as deamidation and sialylation, respectively, result in an increase in the net negative charge on the antibodies and cause a decrease in p*I* values, thereby leading to formation of acidic variants. C-terminal lysine cleavage results in the loss of net positive charge and leads to acidic variant formation. Another mechanism for generating acidic variants is the formation of various types of covalent adducts, e.g. glycation, where glucose or lactose can react with the primary amine of a lysine residue during manufacturing in glucose-rich culture media or during storage if a reducing sugar is present in the formulation. MAbs. 2010 Nov-Dec; 2(6): 613-624*.*

The term "acidic variant" does not include process-related impurities.

The term "process-related impurity," as used herein, refers to impurities that are present in a composition comprising a protein but are not derived from the protein itself.

Process-related impurities include, but are not limited to, host cell proteins (HCPs), host cell nucleic acids, chromatographic materials, and media components.

The term used "Size variants" refers to LMW, HMW or aggregates.

The term used "Low molecular weight (LMW) species" which is a protein backbone-truncated fragments & considered as product-related impurities that contribute to the size heterogeneity of antibody. LMW species often have low or substantially reduced activity relative to the monomeric form of the antibody and can lead to immunogenicity or potentially impact pharmacokinetic properties in vivo. As a result, LMW species are considered critical quality attributes that are routinely monitored during drug development and as part of release testing of purified drug product during manufacturing.

The term used "high molecular weight or HMW "is product-related impurities that contribute to the size heterogeneity of antibody products. The formation of HMW species within a therapeutic antibody drug product as a result of protein aggregation can potentially compromise both drug efficacy and safety (e.g. eliciting unwanted immunogenic response). HMW is considered critical quality attributes that are routinely monitored during drug development and as part of release testing of purified drug product during manufacturing.

The term used "aggregates" are classified based on types of interactions and solubility. Soluble aggregates are invisible particles and cannot be removed with a filter. Insoluble aggregates can be removed by filtration and are often visible to the human eye. Both types of aggregates cause problems in biopharma development. Covalent aggregates arise from the formation of a covalent bond between multiple monomers of a given peptide. Disulfide bond formation of free thiols is a common mechanism for covalent aggregation. Oxidation of tyrosine residues can lead to formation of bityrosine which often results in aggregation. Reversible protein aggregation typically results from weaker protein interactions they include dimers, trimers, multimers among others.

The term used "basic variant" refers to variants can result from the presence of C-terminal lysine or glycine, amidation, succinimide formation, amino acid oxidation or removal of sialic acid, which introduce additional positive charges or removal of negative charges; both types of modifications cause an increase in p*I* values.

The term used "stability" or like terms denotes the tendency of the Omalizumab monomer to undergo a variety of undesired transformations during storage. Such transformations include the formation of oligomers and high molecular weight aggregate(s) (hereinafter terms "aggregate(s)" in which multiple copies of the essentially intact Omalizumab monomer become irreversibly associated with one another through a variety of non-covalent attractions (e.g., electrostatic interactions.) Undesired transformations during storage may also include degradation of the Omalizumab monomer to smaller fragments and/or clipped species. Ideally, a formulation of Omalizumab should minimize, to the greatest extent possible, the tendency of the formulation to result, during storage, in the formation of aggregates, misfolded protein, oligomers, charge varaints and/or fragments of Omalizumab. An important benefit resulting from the ability to reduce formation of unwanted aggregates or fragments is a reduction in the potential toxicity and/or immunogenicity of the drug.

The term "stable" or "stabilized" with respect to long-term storage is understood to mean that Omalizumab contained in the pharmaceutical compositions does not lose more than 20%, or more preferably 15%, or even more preferably 10%, and most preferably 5% of its activity relative to activity of the composition at the beginning of storage.

The term "formulation" refers to a mixture that usually contains a carrier, such as a pharmaceutically acceptable carrier or excipient that is conventional in the art and which is suitable for administration into a subject for therapeutic, diagnostic, or prophylactic purposes. It may include Omalizumab or polypeptide which is derived from the cells in the culture medium.

The term "pharmaceutically acceptable carrier" refers to a non-toxic solid, semisolid or liquid filler, diluent, encapsulating material, formulation auxiliary, or excipient of any conventional type. A pharmaceutically acceptable carrier is non-toxic to recipients at the dosages and concentrations employed and is compatible with other ingredients of the formulation.

The term "pharmaceutical formulation" and "composition" are used interchangeably.

The term "aggregation inhibitor" refers to excipient which prevent aggregation of anti-IgE antibody such as Omalizumab. Aggregation inhibitor isgenerally useful to stabilize when used in high concentration in the formulation. The suitable aggregation inhibitor is arginine.

However, the selection of aggregation inhibitor in combination with suitable excipients such as buffer, surfactant and pH provides desirable result. The present invention provides desired result when arginine or it's salt like arginine HCl is used with phosphate buffer and poloxamer 188 at pH 6.0. Alternatively, the description provides desired result when lysine or it's salt like lysine HCl is used with phosphate buffer or histidine buffer and poloxamer 188 at pH 6.0 (not in the scope of the claims).

The term "aggregation inhibitor" and "stabilizer" are used interchangeably.

The present invention provides novel liquid formulations comprising high concentrated pharmacologically active antibody.

The present invention provides novel and improved liquid formulations which can optionally be lyophilized, comprising of high concentration of therapeutic protein(s), preferably monoclonal antibodies, in suitable buffer(s), one or more suitable aggregation inhibitor, and other excipients which are optionally selected from suitable surfactants and tonicity agents. The stable formulation provides desirable viscosity and prevents formation of aggregates of protein.

In certain embodiment the novel formulations of the present are suitable for subcutaneous administration. The novel formulations of the present invention are stable and avoid or reduce precipitation or aggregation of pharmacologically active antibody.

In certain embodiment the novel formulations of the present invention are stable and avoid or reduce fragmentation or LMW formation of pharmacologically active antibody.

In certain embodiment the novel formulations of the present invention are stable and maintain desire charge heterogeneity.

In certain embodiment the present invention is stable at room temperature. In certain embodiment the novel formulations of the present invention are stable at 2°C to 8°C. In certain embodiment the present formulation is stable at 37°C for at least 15 days or preferably 30 days.

In certain embodiment the novel formulations of the present invention optionally comprise Ca+2 or Mg+2 salt. Optionally the novel formulation of the present invention comprises additives.

The pharmacologically active antibody of the invention is omalizumab.

The novel formulations of the present invention are mainly useful for the treatment of, but not limited to, Asthma, allergy and Chronic Idiopathic Urticaria.

The pharmacologically active antibody is present in high concentration of at least 100 mg/ml. In certain embodiments the pharmacologically active antibody is present in high concentration selected from 100 mg/ml to 200 mg/ml. In certain embodiment the pharmacologically active antibody is present in high concentration selected from 125 mg/ml to 200 mg/ml. In certain embodiment the pharmacologically active antibody is present in high concentration selected from 150 mg/ml.

The invention provides a novel stable formulation comprises a suitable buffer which is phosphate buffer.

In certain embodiment the invention provides a novel stable formulation comprises more than one buffer.

As disclosed herein, which is not present in the wording of the claims, histidine can also be used in its salt form such as histidine hydrochloride when Lysine or Lysine HCl is used as an aggregation inhibitor.

In certain embodiment the buffer is present in the concentration of at least 1mg/ml. In certain embodiment the buffer is present in the range of 1mg/ml to 20mg/ml. In certain embodiment the buffer is present in the range of 1mg/ml to 10mg/ml. In certain embodiment the buffer is present in the range of 1 mg/ml to 5 mg/ml. In certain embodiment the buffer is present in the concentration of 4 mg/ml. In certain embodiment the buffer is present in the concentration of 3.7mg/ml.

In certain embodiment the buffer is present in the concentration of at least 5mM. In certain embodiment the buffer is presented in the range of 5mM to 15mM.

The aggregation inhibitor used in the formulation is selected from arginine or suitable salt thereof.

In certain embodiment the aggregation inhibitors are present in the concentration of at least 1 mg/ml. In certain embodiment the aggregation inhibitors are present in the range of 1 mg/ml to 75 mg/ml. In certain embodiment the aggregation inhibitors are present in the range of 10 mg/ml to 50mg/ml. In certain embodiment the aggregation inhibitors are present in range of 20mg/ml to 50mg/ml. In certain embodiment the aggregation inhibitors are present in range of 30mg/ml to 45mg/ml.

In an embodiment the aggregation inhibitors are present in the range of 40mg/ml.

The aggregation inhibitors are used in the concentration of at least 1mM. In certain embodiment the aggregation inhibitors are present in the range of 1 mM to 200 mM. In certain embodiment the aggregation inhibitors are present in the range of 1 mM to 150 mM. In certain embodiment the aggregation inhibitors are present in the range of 1 mM to 100 mM. In preferred embodiment the aggregation inhibitor amount is 200mM.

The surfactant is poloxamer 188.

As disclosed herein, which is not present in the wording of the claims, the surfactant may be selected from different grades of polysorbate such as but not limited to polysorbate 20, polysorbate 40, polysorbate 60, polysorbate 80 or mixture thereof can be used.

In certain embodiment the surfactant is present in the amount from 0.01% to 0.20%. In certain embodiment the surfactant is present in the amount from 0.02% to 0.04%. In embodiment the surfactant amount is 0.04%. In certain embodiment the surfactant is present in the amount from 0.1mg/ml to 0.5mg/ml. In an embodiment the surfactant amount is about 0.4 mg/ml. In certain embodiment the Ca+2 or Mg+2 salt is used to achieve desired osmolality. In certain embodiment the Ca+2 or Mg+2 salt according to present invention is calcium chloride or Magnesium chloride.

Additives used in the present invention are selected from sodium chloride, mannitol, sucrose, proline, glycine, sodium acetate, sodium citrate, sodium succinate, sodium phosphate and sodium sulfate.

In embodiment the novel formulations of the present invention have pH 6.0 to 7.0, such as a pH of 6.0. In certain embodiments the stable pharmaceutical novel liquid formulation has viscosity of at least 5 mPa·s (5 cp). In certain embodiment the stable pharmaceutical novel liquid formulations have viscosity of 10 mPa·s (10 cps) to 20 mPa·s (20 cps). In certain embodiment the stable pharmaceutical novel liquid formulations have viscosity of 10 mPa·s (10 cps) to 15 mPa·s (15 cps). In certain embodiment the stable pharmaceutical novel liquid formulations have viscosity of 10 mPa·s (10 cps) to 12 mPa·s (12 cps). In certain embodiment the formulation has viscosity of 10 mPa·s (10 cp).

In one aspect of such embodiment the viscosity is selected form 10 mPa·s (10cp), 11 mPa·s (11cp), 12 mPa·s (12cp), 13 mPa·s (13cp), 14 mPa·s (14cp), 15 mPa·s (15cp), 16 mPa·s (16cp), 17 mPa·s (17cp), 18 mPa·s (18cp), 19 mPa·s (19cp) and 20 mPa·s (20cp).

It is understood that the value of viscosity is dependent on the conditions under which the measurement was taken, such as temperature, the rate of shear and the shear stress employed. The apparent viscosity is defined as the ratio of the shear stress to the rate of shear applied. There are a number of alternative methods for measuring apparent viscosity. For example, viscosity can be tested by a suitable cone and plate, parallel plate or other type of viscometer or rheometer.

In certain embodiment the stable pharmaceutical novel liquid formulations have osmolality selected from 0.350 to 0.410 mol·kg⁻¹ (350 to 410 mOsm). In certain embodiment the stable pharmaceutical novel liquid formulations have osmolality selected from 0.370 to 0.410 mol·kg⁻¹ (370 to 410 mOsm). In certain embodiment the stable pharmaceutical novel liquid formulations have osmolality selected from 0.390 to 0.410 mol·kg⁻¹ (390 to 410 mOsm). In certain embodiment the stable pharmaceutical novel liquid formulations have osmolality selected from 0.400 to 0.410 mol·kg⁻¹ (400 to 410 mOsm).

The invention provides a cumulative effect of poloxamer, buffer and aggregation inhibitor.

The present invention provides the pharmaceutical stable liquid formulation comprising:
a. pharmacologically active antibody which binds to IgE;
b. phosphate buffer;
c. suitable aggregation inhibitor selected from Arginine or suitable salt thereof;
d. suitable surfactant and
e. pH 6.0 to 7.0;

wherein the antibody concentration is at least 100 mg/ml,
wherein the pharmacologically active antibody which binds to IgE is Omalizumab, and
wherein the surfactant is Poloxamer 188.

The pharmacologically active antibody which binds to IgE is omalizumab.

In one aspect of such embodiment the formulation is free of histidine buffer.

In another aspect of such embodiment the formulation has high monomer and low aggregation and LMW.

In an embodiment the invention provides the pharmaceutical stable liquid formulation comprising:
a. 125mg/ml to about 160mg/ml of Omalizumab;
b. phosphate buffer;
c. suitable aggregation inhibitor selected from Arginine or suitable salt thereof;
d. poloxamer 188 and e. pH 6.0 to 7.0.

In an embodiment the invention provides the pharmaceutical stable liquid formulation comprising:
a. 150 mg/ml to about 160mg/ml of Omalizumab;
b. phosphate buffer;
c. suitable aggregation inhibitor selected from Arginine or suitable salt thereof;
d. poloxamer 188 and
e. pH 6.0 to 7.0

Present disclosure provides the pharmaceutical stable liquid formulation comprising (not in the scope of the claims):
a. Omalizumab;
b. phosphate buffer;
c. suitable aggregation inhibitor selected from Arginine or suitable salt thereof; wherein the aggregation inhibitor is at concentration of from about 1mM to about 200mM.
d. poloxamer 188 and
e. pH 6.0 to 7.0.

Present disclosure provides the pharmaceutical stable liquid formulation comprising (not in the scope of the claims):
a. Omalizumab;
b. 5mg/ml to about 20mg/ml of phosphate buffer;
c. suitable aggregation inhibitor selected from Arginine or suitable salt thereof; wherein the aggregation inhibitor is at concentration of from about 1mM to about 200mM.
d. poloxamer 188 and
e. pH 6.0 to 7.0

Present disclosure provides the pharmaceutical stable liquid formulation comprising (not in the scope of the claims):
a. Omalizumab;
b. 5mg/ml to about 20mg/ml of phosphate buffer;
c. suitable aggregation inhibitor selected from Arginine or suitable salt thereof; wherein the aggregation inhibitor is at concentration of from about 1mM to about 200mM.
d. 0.02% to about 0.04% of poloxamer 188 and
e. pH 6.0 to 7.0

Present disclosure provides the pharmaceutical stable liquid formulation comprising (not in the scope of the claims):
a. 100 to 160 mg/ml of Omalizumab antibody;
b. 5mM to 20mM phosphate buffer;
c. 100mM to about 200mM of Lysine as an aggregation inhibitor;
d. 0.02% to about 0.04% of Poloxamer 188
e. pH 6.0

Present disclosure provides the pharmaceutical stable liquid formulation comprising (not in the scope of the claims):
a. about 150 mg/ml of Omalizumab antibody;
b. about 20mM phosphate buffer;
c. about 200mM of Lysine or Lysine HCl as an aggregation inhibitor;
d. about 0.04% of Poloxamer 188
e. pH 6.0

In this example, the formulation is free of arginine.

In another embodiment the invention provides the pharmaceutical stable liquid formulation comprising:
a. 100 to 160 mg/ml of Omalizumab antibody;
b. 5mM to 20mM phosphate buffer;
c. 100mM to about 200mM of Arginine or Arginine HCl as an aggregation inhibitor;
d. 0.02% to about 0.04% of Poloxamer 188
e. pH 6.0

In another embodiment the invention provides the pharmaceutical stable liquid formulation comprising:
a. about 150 mg/ml of Omalizumab antibody;
b. about 20mM phosphate buffer;
c. about 200mM of Arginine or Arginine HCl as an aggregation inhibitor;
d. about 0.04% of Poloxamer 188
e. pH 6.0

In such embodiment the invention provides a high amount of monomer which is at least more than 90% and low amount of aggregates, HMW and LMW. In certain embodiment of such formulation further provides desirable charge variants where acidic variants are below 20%. In certain embodiment of such formulation further provides desirable charge variants where basic variants are below 25%. In certain embodiment of such formulation viscosity is below 15 mPa·s (15cp). In certain embodiment of such formulation osmolality is about 0.390 to 0.410 mol·kg⁻¹ (390 to 410mOsm).

Suitably, the liquid pharmaceutical compositions of the invention have a shelf life of at least 3 months, suitably at least 6 months, suitably at least 12 months, more suitably at least 24 months at a temperature of 2-8° C.

Suitably, the liquid pharmaceutical compositions of the invention have a shelf life of at least 7 days, suitably at least 15 days, suitably 1 months at a temperature of 37° C.

In one example, which is not present in the wording of the claims, is a pharmaceutical stable formulation comprising:
a. high concentration of pharmacologically active anti-IgE antibody in an amount of about 50mg/ml to 150 mg/ml;
b. suitable buffer comprising Histidine or Histidine HCl in an amount of at least about 8 mM or 20mM;
c. suitable aggregation inhibitor Lysine or Lysine HCL;
d. Polysorbate or poloxamer 188 and
e. pH 6.0 to 7.0

In an embodiment suitable additive comprising NaCl, Mannitol, Sucrose, Proline, and Glycine.

In one example, which is not present in the wording of the claims, is a pharmaceutical stable formulation comprising;
a. high concentration of pharmacologically active antibody;
b. suitable buffer is methionine;
c. suitable aggregation inhibitor Arginine or Arginine HCl;
d. Polysorbate or poloxamer 188 and
e. pH 6.0 to 7.0

In one example, which is not present in the wording of the claims, is a pharmaceutical stable formulation comprising;
a. high concentration of pharmacologically active antibody;
b. suitable buffer is methionine and phosphate;
c. suitable aggregation inhibitor Arginine or Arginine HCl;
d. Poloxamer 188 and
e. pH 6.0 to 7.0

In one example, which is not present in the wording of the claims, is a pharmaceutical stable formulation comprising;
a. high concentration of pharmacologically active antibody;
b. suitable buffer is histidine;
c. suitable aggregation inhibitor is lysine or lysine HCl;
d. Poloxamer 188 and
e. pH 6.0 to 7.0

In one example, which is not present in the wording of the claims, is a pharmaceutical stable formulation comprising;
a. 100 to 160 mg/ml of Omalizumab antibody;
b. 5mM to 20mM histidine buffer;
c. 100mM to about 200mM of Lysine or Lysine HCl as an aggregation inhibitor;
d. 0.02% to about 0.04% of Poloxamer 188;
e. pH 6.0

In one example, which is not present in the wording of the claims, is a pharmaceutical stable formulation comprising;
a. about 150 mg/ml of Omalizumab antibody;
b. about 20mM histidine buffer;
c. about 200mM of Lysine or Lysine HCl as an aggregation inhibitor;
d. about 0.04% of Poloxamer 188;
e. pH 6.0

In the above examples, the pharmaceutically stable formulation which is free of Arginine. Invention further provides a high amount of monomer which is at least more than 90% and low amount of aggregates, HMW and LMW. In certain embodiment of such formulation further provides desirable charge variants where acidic variants are below 20%. In certain embodiment of such formulation further provides desirable charge variants where basic variants are below 25%. In certain embodiment of such formulation viscosity is below 15 mPa·s (15cp). In certain embodiment of such formulation osmolality is about 0.390 to 0.410 mol·kg⁻¹ (390 to 410mOsm).

Further, formulation was also tried to prepare with addition of 20 mM of phosphate buffer, 200 mM of Glycine HCl and Poloxamer 188 at pH 6.0. However, during concentrating of the bulk solution, the viscosity of the formulation was increased a lot and it was unable to achieve the Omalizumab concentration to desired concentration i.e. between 145 to 155 mg/mL. So, this formulation was not incubated for stability study.

In a further aspect, the present invention provides a drug delivery device comprising a liquid pharmaceutical composition as defined herein. Preferably the drug delivery device comprises a chamber within which the pharmaceutical composition resides. More preferably, the drug delivery device is sterile.

The drug delivery device may be a vial, ampoule, syringe, injection pen, autoinjector (e.g. essentially incorporating a syringe). When the drug delivery device is a syringe, it is preferably an injection pen. Wherein the syringe is a glass or plastic syringe.

In yet a further aspect, the present invention provides a kit of parts comprising a drug delivery device (without the liquid pharmaceutical composition incorporated therein), a liquid pharmaceutical composition as defined herein (optionally contained in a separate package or container), and optionally a set of instructions with directions regarding the administration (e.g. sub-cutaneous or intravenous) of the liquid pharmaceutical composition. The user may then fill the drug delivery device with the liquid pharmaceutical composition (which may be provided in a vial or ampoule or such like) prior to administration.

Also described, package is comprising a liquid pharmaceutical composition as defined herein. Suitably the package comprises a drug delivery device as defined herein, suitably a plurality of drug delivery devices. The package may comprise any suitable container for containing one or more drug delivery devices.

The liquid pharmaceutical compositions defined herein may be used to treat any one or more of the aforementioned diseases or medical disorders. In a particular embodiment, the liquid pharmaceutical compositions are used to treat Asthma, Urticaria, allergy, Chronic idiopathic urticaria.

The liquid pharmaceutical compositions are suitably parenterally administered, either via intravenous injection or via sub-cutaneous injection preferably sub-cutaneous injection.

The present invention provides examples below for illustrative purpose and scope of invention is not limited with illustrative examples.

### EXAMPLE 1

Omalizumab injectable formulation was formulated with 150 mg/mL antibody concentration for subcutaneous administration.

Formulation was prepared with addition of 20 mM of phosphate buffer, 200 mM of Arginine HCl and Poloxamer 188 at pH 6.0. The bulk solution was filtrated with 0.2µm PVDF filter to get the filtrated solution and filled 1 mL of filtered solution in 1 mL glass PFS. The composition of formulation is given below:

**TABLE 1**

| **Component** | **Amount** |
|---|---|
| Omalizumab | 150 mg/mL |
| Arginine HCl | 42.1 mg/mL (200 mM) |
| Sodium phosphate monobasic monohydrate | 2.07 mg/mL (15 mM) |
| Sodium phosphate dibasic heptahydrate | 1.34 mg/mL (5 mM) |
| Poloxamer 188 | 0.4 mg/mL |

The stability study was executed at 37°C for 1 month and below is the summary of data analysed:

**TABLE 2**

| **Analytical tests** | **Results** | |
|---|---|---|
| | 0 Day | 1 Month |
| % Monomer by SEC | 99.56 | 98.08 |
| % High molecular weight (HMW) species by SEC | 0.03 | 0.36 |
| % Low molecular weight (LMW) species by SEC | 0.42 | 1.24 |
| % Acidic variant by CEX | 7.43 | 15.75 |
| % Basic variant by CEX | 9.73 | 21.09 |

Based on the above results, the formulation was stable for 1 month stored at 37°C as % monomer and % HMW found at 98.08 % & 0.36 % respectively after 1 month.

### REFERENCE EXAMPLE 2

Omalizumab injectable formulation was formulated with 150 mg/mL antibody concentration for subcutaneous administration.

Formulation was prepared with addition of 20 mM of phosphate buffer, 200 mM of Lysine HCl and Poloxamer 188 at pH 6.0. The bulk solution was filtrated with 0.2µm PVDF filter to get the filtrated solution and filled 1 mL of filtered solution in 1 mL glass PFS. The composition of formulation is given below:

**TABLE 3**

| **Component** | **Amount** |
|---|---|
| Omalizumab | 150 mg/mL |
| Lysine HCl | 36.40 mg/mL (200 mM) |
| Sodium phosphate monobasic monohydrate | 2.07 mg/mL (15 mM) |
| Sodium phosphate dibasic heptahydrate | 1.34 mg/mL (5 mm) |
| Poloxamer 188 | 0.4 mg/mL |

The stability study was executed at 37°C for 1 month and below is the summary of data analysed:

**TABLE 4**

| **Analytical tests** | **Results** | |
|---|---|---|
| | 0 Day | 1 Month |
| % Monomer by SEC | 99.48 | 98.39 |
| % High molecular weight (HMW) species by SEC | 0.03 | 0.36 |
| % Low molecular weight (LMW) species by SEC | 0.47 | 1.25 |
| % Acidic variant by CEX | 7.64 | 15.87 |
| % Basic variant by CEX | 7.80 | 19.80 |

Based on the above results, the formulation was stable for 1 month stored at 37°C as % monomer and % HMW found at 98.39 % & 0.36 % respectively after 1 month.

### REFERENCE EXAMPLE 3

Omalizumab injectable formulation was formulated with 150 mg/mL antibody concentration for subcutaneous administration.

Formulation was prepared with addition of 20 mM of Histidine buffer, 200 mM of Lysine HCl and Poloxamer 188 at pH 6.0. The bulk solution was filtrated with 0.2µm PVDF filter to get the filtrated solution and filled 1 mL of filtered solution in 1 mL glass PFS. The composition of formulation is given below:

**TABLE 5**

| **Component** | **Amount** |
|---|---|
| Omalizumab | 150 mg/mL |
| Lysine HCl | 36.40 mg/mL (200 mM) |
| L - Histidine | 1.37 (11 mM) |
| L - Histidine HCl monohydrate | 2.34 (9 mM) |
| Poloxamer 188 | 0.4 mg/mL |

The stability study was executed at 37°C for 1 month and below is the summary of data analysed:

**TABLE 6**

| **Analytical tests** | **Results** | |
|---|---|---|
| | 0 Day | 1 Month |
| % Monomer by SEC | 99.58 | 98.09 |
| % High molecular weight (HMW) species by SEC | 0.03 | 0.35 |
| % Low molecular weight (LMW) species by SEC | 0.39 | 1.56 |
| % Acidic variant by CEX | 7.41 | 14.69 |
| % Basic variant by CEX | 9.73 | 20.99 |

Based on the above results, the formulation was stable for 1 month stored at 37°C as % monomer and % HMW found at 98.09 % & 0.35 % respectively after 1 month.

### EXAMPLE 4

The stability study of above three formulations (Example 1 to 3) have been performed at refrigerator (2°C to 8°C) for 3 months. The summary of the data given in the below table 7:

**TABLE 7**

| **Name** | % HMW by SEC | % LMW by SEC | % Monomer by SEC | % Acidic variants by CEX | % Basic variants by CEX |
|---|---|---|---|---|---|
| Example 1 | 0.17 | 0.24 | 99.59 | 9.62 | 8.06 |
| Reference Example 2 | 0.19 | 0.34 | 99.47 | 9.58 | 7.9 |
| Reference Example 3 | 0.18 | 0.36 | 99.46 | 9.22 | 7.04 |

All three formulations (Examples 1 to 3) were found stable after 3 months storage at 2°C to 8°C. All three formulations show high % of monomer which were measured by SEC-HPLC. In addition, HMW, LMW and charge variants are also very low after storing 3 months which makes the formulation highly stable for longer time.

All three formulations were charged for long term stability which is ongoing.

### EXAMPLE 5

All three formulations described in Example 1, Reference Example 2 and Reference Example 3 are further evaluated through Differential Scanning Fluorimetry (DSF) to analyse protein folding state and thermal stability. It is a fast, reliable and robust tool to examine protein stability, thermal protein unfolding by controlled heating (e.g. 0.5 °C / min) of protein in a range of increasing temperature (e.g. 25°C to 95°C). Protein concentrations of samples were normalized before analysis.

Examples 1 to 3 were used to perform this study along with F1 formulation which comprises 150 mg/ml Omalizumab formulated in 200 mM Arginine HCl, 20 mM Histidine buffer and 0.4 mg/ml Polysorbate 20. The data are summarized in table 8:

**TABLE 8**

| **Name** | **Onset temperature** | **Tm1** | **Tm2** | **Tm3** |
|---|---|---|---|---|
| F1 | 58.4 | 65.1 | 74.7 | 78.6 |
| Example 1 | 62.2 | 68.7 | 77.5 | 81.5 |
| Reference Example 2 | 61.2 | 68.0 | 77.0 | 81.1 |
| Reference Example 3 | 60.1 | 66.3 | 75.8 | 80.2 |

Base on the above data, it is evident that onset temperature of all three formulations (Example 1 to 3) is higher than F1 formulation which indicated that the all three formulations (Example 1 to 3) were more thermally stable than F1 formulation.

### EXAMPLE 6

Formulation was prepared with addition of 20 mM of Histidine buffer, 200 mM of Arginine HCl and Poloxamer 188 at pH 6.0. The bulk solution was filtrated with 0.2µm PVDF filter to get the filtrated solution and filled 1 mL of filtered solution in 1 mL glass PFS. The formulation comprising polysorbate 20 is not in the scope of the claims.

Effect of Polysorbate 20 and Poloxamer 188 was evaluated on stability of formulations. Below compositions were used for this study:

**TABLE 9**

| **Component** | **Amount (mg/mL)** | **Amount (mg/mL)** |
|---|---|---|
| Omalizumab | 150 | 150 |
| Arginine HCl | 42.10 | 42.10 |
| L - Histidine | 1.37 | 1.37 |
| L - Histidine HCl monohydrate | 2.34 | 2.34 |
| Poloxamer 188 | 0.4 | - |
| Polysorbate 20 | - | 0.4 |

Both formulations were charged under stability at 37°C for 15 days and below are the results:

**TABLE 10**

| **Analytical tests** | **Results with Poloxamer 18** | | **Results with Polysorbate 20** | |
|---|---|---|---|---|
| | 0 Day | 15 days | 0 Day | 15 days |
| % High molecular weight (HMW) species by SEC | 0.06 | 0.49 | 0.06 | 0.62 |
| % Monomer by SEC | 99.65 | 98.54 | 99.65 | 98.43 |

Table 10 shows that % HMW was found higher with Polysorbate 20 than Poloxamer 188 after 15 days incubated at 37°C.

### EXAMPLE 7

F2 Formulation was prepared with addition of 20 mM of Histidine buffer, 200 mM of Arginine HCl and Poloxamer 188 at pH 6.0. The bulk solution was filtrated with 0.2µm PVDF filter to get the filtrated solution and filled 1 mL of filtered solution in 1 mL glass PFS. Formulation F2 is not in the scope of the claims.

The stability of F2 formulation was evaluated with Example 1 to 3 for 1 month charged at 37°C. Below are the summarized data of all formulations:

**TABLE 11**

| **Name** | % HMW by SEC | % LMW by SEC | % Monomer by SEC |
|---|---|---|---|
| F2 | 0.37 | 2.04 | 97.59 |
| Example 1 | 0.36 | 1.24 | 98.04 |
| Reference Example 2 | 0.36 | 1.25 | 98.39 |
| Reference Example 3 | 0.35 | 1.56 | 98.09 |

As shown effect of Poloxamer 188 over Polysorbate 20 in example 6, we have further evaluated combined effect of Poloxamer with aggregation inhibitor and buffer. It is evident from table 11 that example 1 and reference example 2 have low HMW and LMW compared to F2 which shows the improved effect of phosphate buffer in combination with Poloxamer 188 used in example 1 and reference example 2.

Reference example 3 has lower HMW and LMW compared to F2 formulation which shows the effect of Lysine and Poloxamer 188 over Arginine and Poloxamer 188 used in F2.

Lower percentage of LMW are also important to formulate stable formulation as higher LMW produces fragmentations of antibody which not only reduce the shelf life of formulation but also make formulation less potent.

## Claims

1. A pharmaceutical stable liquid formulation comprising:
a. pharmacologically active antibody which binds to IgE;
b. phosphate buffer;
c. suitable aggregation inhibitor selected from Arginine or suitable salt thereof;
d. suitable surfactant and
e. pH 6.0 to 7.0
wherein the antibody concentration is at least 100 mg/ml,
wherein the pharmacologically active antibody which binds to IgE is Omalizumab, and
wherein the surfactant is Poloxamer 188.

2. The pharmaceutical formulation according to claim 1 wherein the Omalizumab is present from at least 100mg/ml to 200mg/ml.

3. The pharmaceutical formulation according to claim 2 wherein the Omalizumab is present from at least 150 mg/ml.

4. The pharmaceutical formulation according to claim 1 wherein the phosphate buffer is present from 5mg/ml to 20mg/ml.

5. The pharmaceutical formulation according to claim 1 wherein the aggregation inhibitor is present from 100 mM to 200 mM.

6. The pharmaceutical formulation according to claim 1 wherein the surfactant is present from 0.02% to 0.04%.

7. The pharmaceutical formulation according to claim 6 wherein the pH is 6.0 .

8. The pharmaceutical liquid formulation according to claim 1 comprising:
a. 100 to 160 mg/ml of Omalizumab antibody;
b. 5mM to 20mM phosphate buffer;
c. 100mM to 200mM of Arginine or Arginine HCl as an aggregation inhibitor; and
d. 0.02% to 0.04% of Poloxamer 188,
e. pH 6.0 .

9. The pharmaceutical liquid formulation according to claim 8 comprising:
a. 150 mg/ml of Omalizumab antibody;
b. 20mM phosphate buffer;
c. 200mM of Arginine or Arginine HCl as an aggregation inhibitor; and
d. 0.04% of Poloxamer 188,
e. pH 6.0 .

10. A drug delivery device comprising the pharmaceutical liquid formulation as claimed in claim 1.

11. The pharmaceutical liquid formulation according to claim 1 for use in the treatment of a disease selected from asthma, urticaria, allergy, or chronic idiopathic urticaria.

12. The pharmaceutical liquid formulation according to claim 1 wherein said formulation has a viscosity from 10 mPa·s (10cp) to 15 mPa·s (15cp).

13. The pharmaceutical liquid formulation according to claim 1 wherein the formulation is stable for at least 1 month when stored at 37°C, and wherein stability is determined by a monomer content of least 90%.

## Patentansprüche

1. Pharmazeutische, stabile, flüssige Formulierung, umfassend:
a. einen pharmakologisch aktiven Antikörper, der an IgE bindet;
b. Phosphatpuffer;
c. einen geeigneten Aggregationshemmer, ausgewählt aus Arginin oder einem geeigneten Salz davon;
d. ein geeignetes Tensid und
e. pH 6,0 bis 7,0,
wobei die Antikörperkonzentration mindestens 100 mg/ml beträgt,
wobei es sich bei dem pharmakologisch aktiven Antikörper, der an IgE bindet, um Omalizumab handelt und
wobei es sich bei dem Tensid um Poloxamer 188 handelt.

2. Pharmazeutische Formulierung nach Anspruch 1, wobei das Omalizumab von mindestens 100 mg/ml bis 200 mg/ml vorhanden ist.

3. Pharmazeutische Formulierung nach Anspruch 2, wobei das Omalizumab ab mindestens 150 mg/ml vorhanden ist.

4. Pharmazeutische Formulierung nach Anspruch 1, wobei der Phosphatpuffer von 5 mg/ml bis 20 mg/ml vorhanden ist.

5. Pharmazeutische Formulierung nach Anspruch 1, wobei der Aggregationshemmer von 100 mM bis 200 mM vorhanden ist.

6. Pharmazeutische Formulierung nach Anspruch 1, wobei das Tensid von 0,02 % bis 0,04 % vorhanden ist.

7. Pharmazeutische Formulierung nach Anspruch 6, wobei der pH-Wert 6,0 beträgt.

8. Pharmazeutische flüssige Formulierung nach Anspruch 1, umfassend:
a. 100 bis 160 mg/ml Omalizumab-Antikörper;
b. 5 mM bis 20 mM Phosphatpuffer;
c. 100 mM bis 200 mM Arginin oder Arginin-HCl als Aggregationshemmer; und
d. 0,02 % bis 0,04 % Poloxamer 188,
e. pH 6,0.

9. Pharmazeutische flüssige Formulierung nach Anspruch 8, umfassend:
a. 150 mg/ml Omalizumab-Antikörper;
b. 20 mM Phosphatpuffer;
c. 200 mM Arginin oder Arginin-HCl als Aggregationshemmer; und
d. 0,04 % Poloxamer 188,
e. pH 6,0.

10. Arzneimittelabgabevorrichtung, umfassend die pharmazeutische flüssige Formulierung nach Anspruch 1.

11. Pharmazeutische flüssige Formulierung nach Anspruch 1 zur Verwendung bei der Behandlung einer Krankheit, ausgewählt aus Asthma, Urtikaria, Allergie oder chronischer idiopathischer Urtikaria.

12. Pharmazeutische flüssige Formulierung nach Anspruch 1, wobei die Formulierung eine Viskosität von 10 mPa·s (10 cp) bis 15 mPa·s (15 cp) aufweist.

13. Pharmazeutische flüssige Formulierung nach Anspruch 1, wobei die Formulierung bei Lagerung bei 37 °C mindestens 1 Monat lang stabil ist und wobei die Stabilität durch einen Monomergehalt von mindestens 90 % bestimmt wird.

## Revendications

1. Formulation liquide stable pharmaceutique comprenant :
a. un anticorps pharmacologiquement actif se liant à l'IgE ;
b. un tampon phosphate ;
c. un inhibiteur d'agrégation approprié choisi parmi l'arginine ou un sel approprié de celle-ci ;
d. un tensioactif approprié et
e. un pH 6,0 à 7,0
dans laquelle la concentration d'anticorps est d'au moins 100 mg/ml,
dans laquelle un anticorps pharmacologiquement actif se liant à l'IgE est l'omalizumab, et
dans laquelle le tensioactif est le Poloxamer 188.

2. Formulation pharmaceutique selon la revendication 1, dans laquelle l'omalizumab est présent à une concentration d'au moins 100 mg/ml à 200 mg/ml.

3. Formulation pharmaceutique selon la revendication 2, dans laquelle l'omalizumab est présent à une concentration d'au moins 150 mg/ml.

4. Formulation pharmaceutique selon la revendication 1, dans laquelle le tampon phosphate est présent à une concentration de 5 mg/ml à 20 mg/ml.

5. Formulation pharmaceutique selon la revendication 1 dans laquelle l'inhibiteur d'agrégation est présent à une concentration de 100 mM à 200 mM.

6. Formulation pharmaceutique selon la revendication 1, dans laquelle le tensioactif est présent à une concentration de 0,02 % à 0,04 %.

7. Formulation pharmaceutique selon la revendication 6 dans laquelle le pH est de 6,0.

8. Formulation liquide pharmaceutique selon la revendication 1 comprenant :
a. 100 à 160 mg/ml d'anticorps d'omalizumab ;
b. 5 mM à 20 mM de tampon phosphate ;
c. 100 mM à 200 mM d'arginine ou de chlorhydrate d'arginine comme inhibiteur d'agrégation ; et
d. 0,02 % à 0,04 % de Poloxamer 188,
e. un pH 6,0.

9. Formulation liquide pharmaceutique selon la revendication 8 comprenant :
a. 150 mg/ml d'anticorps d'omalizumab ;
b. 20 mM de tampon phosphate ;
c. 200 mM d'arginine ou de chlorhydrate d'arginine comme inhibiteur d'agrégation ; et
d. 0,04 % de Poloxamer 188,
e. un pH 6,0.

10. Dispositif d'administration de médicament comprenant la formulation liquide pharmaceutique selon la revendication 1.

11. Formulation liquide pharmaceutique selon la revendication 1 destinée à être utilisée dans le traitement d'une maladie choisie parmi l'asthme, l'urticaire, l'allergie ou l'urticaire chronique idiopathique.

12. Formulation liquide pharmaceutique selon la revendication 1, dans laquelle ladite formulation présente une viscosité de 10 mPa·s (10 cp) à 15 mPa·s (15 cp).

13. Formulation liquide pharmaceutique selon la revendication 1, dans laquelle la formulation est stable pendant au moins 1 mois lorsqu'elle est stockée à 37 °C, et dans laquelle la stabilité est déterminée par une teneur en monomère d'au moins 90 %.
